# EUROPÄISCHE PATENTSCHRIFT

(11) **EP 0 513 565 B1**
(45) Veröffentlichungstag und Bekanntmachung des Hinweises auf die Patenterteilung: **16.10.1996**
(21) Anmeldenummer: 92106829.2
(22) Anmeldetag: 22.04.1992
(51) Int. Cl.: C12P 21/08, G01N 33/577, C07K 2/00

(54) **Monoklonale Antikörper gegen den Plasmin-Antiplasmin Komplex, Verfahren zu ihrer Herstellung sowie ihre Verwendung**
Monoclonal antibodies against the plasmin-antiplasmin complex, a method for the preparation thereof and the use thereof
Anticorps monoclonaux contre le complexe plasmine-antiplasmine, procédé pour leur préparation et leur utilisation

(30) Priorität: 16.05.1991 DE 4115993
(43) Veröffentlichungstag der Anmeldung: 19.11.1992
(73) Patentinhaber: BEHRINGWERKE Aktiengesellschaft, 35001 Marburg (DE)
(72) Erfinder: Hock, Johann, W-3550 Marburg (DE); Pelzer, Hermann, Florida 33176 (US)

(56) Entgegenhaltungen:
- EP-A- 0 159 025
- BE-A- 896 543
- PROCEEDINGS OF THE NATIONAL ACADEMY OF SCIENCES OF USA. Bd. 87, Februar 1990, WASHINGTON US Seiten 1114 - 1118; GUY L. REED ET AL.

## Beschreibung

Die Erfindung betrifft monoklonale Antikörper (MAK) und deren Fragmente, die eine spezifische Affinität zum Plasmin-Antiplasmin (Pl-AP) Komplex haben und die keine oder nur eine sehr geringe Affinität zu den freien Einzelkomponenten dieses Komplexes aufweisen und ihre Verwendung in Diagnostika, als Wirkstoff oder Wirkstoffträger sowie Hybridomazellinien, die solche Antikörper produzieren.

Das fibrinolytische System, bestehend aus Plasminogen, Plasminogen-Aktivatoren sowie deren Inhibitoren, spielt eine wichtige Rolle unter anderem bei der Auflösung von Blutgerinnseln. Endprodukt einer kaskadenartigen Aktivierungskette ist die Serinprotease Plasmin, die unlösliche Fibringerinnsel in lösliche Fibrin-Abbauprodukte spalten kann und somit eine überschießende Thrombusbildung verhindert. Die Wirkung von Plasmin wird durch den Proteaseninhibitor α2-Antiplasmin kontrolliert, der mit Plasmin unter Bildung eines kovalenten Enzym-Inhibitor-Komplexes reagiert und Plasmin dadurch inaktiviert. Die Konzentration dieses Komplexes im Plasma gibt Auskunft über das Ausmaß der Aktivierung des fibrinolytischen Systems und ist damit geeignet, Störungen dieses Systems zu entdecken.

Bekannt ist die Bestimmung der Konzentration des Komplexes mit einem Sandwich-ELISA, bei dem ein erster ("Fänger")-Antikörper gegen α2-Antiplasmin als Beschichtungsantikörper fungiert, während ein zweiter, enzymmarkierter Antikörper gegen Plasmin zur Detektion des an den Fängerantikörper gebundenen Komplexes dient (Harpel,PC (1981) J. Clin. Invest. 68: 46-55; Holvoet,P et al. (1986) Thromb.Haemostas. 56: 124-127). Eine weitere Methode zur Bestimmung des Plasmin-α2-Antiplasmin-Komplexes mit einem immobilisierten monoklonalen Fänger-Antikörper gegen Plasminogen und einem Peroxidasemarkierten Detektionsantikörper gegen α2-Antiplasmin ist ebenfalls beschrieben (Mimuro et al. (1987) Blood 69: 446-453).

Der Nachteil der genannten Testsysteme liegt darin, daß der Enzym-Inhibitor-Komplex mit dem in der Probe normalerweise in großem Überschuß vorliegenden freien Inhibitor bzw. Proenzym um die Bindung an den Fänger-Antikörper konkurriert. Die Folge ist, daß die Menge des gebundenen und damit der Bestimmung zugänglichen Komplexes von der Konzentration der freien Einzelkomponenten des Komplexes abhängig ist. Eine Lösung dieses Problems wird in allen beschriebenen Methoden dadurch erreicht, daß die zur Testung eingesetzten Proben sehr stark verdünnt werden. Dabei muß ein erheblicher Verlust an Sensitivität des Tests in Kauf genommen werden (siehe auch Tabelle 2).

Obwohl das generelle Verfahren zur Herstellung von monoklonalen Antikörpern seit 1975 (KÖHLER und MILSTEIN, Nature, Vol. 256, S. 495 (1975)) bekannt war, bestanden immer noch große Schwierigkeiten, einen Antikörper zu produzieren, der die gewünschten Eigenschaften besitzt, das heißt, der selektiv den Pl-AP Komplex erkennt. Entscheidend für die Verwendbarkeit eines solchen Antikörpers in einem spezifischen diagnostischen Verfahren oder als Therapeutikum ist die geringe oder möglichst nicht vorhandene Affinität zu Plasminogen oder Antiplasmin in nicht komplexiertem Zustand.

Aufgabe der Erfindung war es daher, einen monospezifischen Antikörper zur Verfügung zu stellen, der selektiv eine spezifische Affinität zu dem Plasmin-Antiplasmin Komplex hat, aber keine oder nur eine sehr geringe Affiinität zu den Einzelkomponenten bzw. Vorläufern Plasminogen und Antiplasmin in nicht-komplexiertem Zustand hat.

Überraschenderweise wurde gefunden, daß sowohl durch Immunisierung mit Plasmin als auch mit α2-Antiplasmin, welches mit Hilfe von Ammoniak aus einem Komplex mit Plasmin freigesetzt wurde, Antikörper der gewünschten Spezifität erhalten werden konnten.

Gegenstand der Erfindung sind somit monospezifische Antikörper gegen den Plasmin-Antiplasmin-Komplex, die eine hohe spezifische Affinität zu dem Komplex aufweisen und keine oder nur eine sehr geringe Affinität zu den freien Einzelkomponenten haben.

Bevorzugt ist dabei ein monospezifischer Antikörper wie vorstehend beschrieben, der von der Hybridomazellinie BW PAP6 produziert wird.

Gegenstand der Erfindung sind ferner monospezifische Antikörper wie vorstehend beschrieben, die an ein Antigen binden, das durch den Referenzantikörper BMA PAP6 gebunden wird.

Besonders bevorzugt ist der MAK BMA PAP6.

Ferner ist Gegenstand der Erfindung ein Antigen, das von einem der oben beschriebenen Antikörper gebunden wird.

Ferner sind Gegenstand der Erfindung Hybridomazellinien, die monoklonale Antikörper wie vorstehend beschrieben produzieren.

Bevorzugt sind dabei Hybridomazellinien, die MAK BMA PAP6 produzieren.

Besonders bevorzugt ist die Hybridomazellinie BW PAP 6.

Gegenstand der Erfindung ist auch die Verwendung eines Antikörpers wie vorstehend beschrieben zur Diagnostik.

Außerdem sind Gegenstand der Erfindung diagnostische Verfahren zum Nachweis des Plasmin-Antiplasmin Komplex, wobei mindestens einer der oben beschriebenen Antikörper als spezifischer Bindungspartner eingesetzt wird.

Bevorzugt sind dabei Verfahren wie vorstehend beschrieben, wobei mindestens einer der spezifischen Bindungspartner mit einer nachweisbaren Markierung versehen ist.

Besonders bevorzugt ist sin solches diagnostisches Verfahren, wobei die Markierung z. B. ein Enzym ist und der andere spezifische Bindungspartner direkt oder indirekt an eine Festphase gebunden wird.

Unter Plasmin-Antiplasmin Komplex werden im Sinne dieser Erfindung alle Plasmin-Antiplasmin Komplexe verstanden, die durch die erfindungsgemäßen Antikörper detektiert werden können.

Die erfindungsgemäßen Antikörper erlauben den Aufbau von Tests zur qantitativen Bestimmung der Konzentration des Pl-AP-Komplexes, beispielsweise in unverdünnten Plasmaproben, unabhängig von den Konzentrationen von freiem Plasminogen und α2-Antiplasmin in der Probe. Die erfindungsgemäßen Antikörper sind monoklonale Antikörper.

Antikörper im Sinne der Erfindung sind auch die dem Fachmann an sich bekannten immunreaktiven Fragmente von Antikörpern. Bevorzugt unter den Fragmenten sind hierbei F(ab')₂ Fragmente.

Ein bevorzugter Antikörper im Sinne dieser Erfindung ist der BMA PAP 6, der von der Hybridomazellinie BW PAP 6 produziert wird. Diese Hybridomazellinie ist unter der Nummer DSM ACC2004 am 21. Februar 1991 bei der "Deutsche Sammlung von Mikroorganismen und Zellkulturen GmbH (DSM)" hinterlegt worden.

Diese Zellinie z. B. ist erhalten worden nach dem Fachmann bekannten Verfahren (siehe z. B. Monoclonal Antibodies, Kennett, R.; Mc Kearn, T. and Bechtol, K.; eds., Plenum Press, 1980, einschließlich der dort enthaltenen Literatur), durch Hybridisierung einer Mausmyelomzellinie mit Milzzellen von Mäusen, die immunisiert wurden mit Plasmin oder α2-Antiplasmin, welches mit Hilfe von Ammoniak, bevorzugterweise 0.1 - 1 % (v/v), aus einem Pl-AP Komplex freigesetzt wurde. Zur Fusion können verschiedene murine (Maus) Myelomzellinien verwendet werden. Einige dieser Zellinien sind in MELCHERS, F.; POTTER, M. und WARNER, N., eds.; Current Topics in Microbiology and Immunology, Vol. 81, Springer Verlag (1978) beschrieben. Die Hybridisierung erfolgt meistens in Gegenwart von Polyethylenglykol als Fusionspromoter, andere Fusionspromotoren wie Sendai-Virus können auch verwendet werden. Das Verhältnis von Milz- zu Myelomzellen kann variieren, gewöhnlich wird ein Verhältnis von 2 - 10:1 verwendet. Nach der Fusion werden die Zellen nach bekannten Verfahren kultiviert und die Zellüberstände auf das Vorhandensein von Antikörpern mit den gewünschten Eigenschaften getestet. Dazu kann zum Beispiel ein Enzymimmunoassay eingesetzt werden wobei eine mit anti-Maus IgG (aus Kaninchen) beschichtete Mikrotitrationsplatte mit Zellüberständen inkubiert wird.

Danach wird a) mit einem Standard Humanplasma und b) mit einem Pl-AP Komplex reichen Plasma inkubiert. In einem weiteren Inkubationsschritt wird ein Gemisch aus Peroxidase markierten Antikörpern gegen Plasmin und Antiplasmin zugesetzt und anschließend die gebundene Peroxidaseaktivität bestimmt. Gegebenenfalls können zwischen den Inkubationsschritten Waschschritte durchgeführt werden. Zellüberstände, die mit b) aber nicht mit a) reagieren, enthalten Antikörper mit den gewünschten Eigenschaften und die entsprechenden Zellkulturen werden zur weiteren Klonierung verwendet.

Größere Mengen der gewünschten, erfindungsgemäßen Antikörper können z. B. durch in-vitro (Zellkulturen) oder in-vivo (Ascites) Züchtung erhalten werden.
Die so erhaltenen Antikörper können durch ihre Immunglobulinklasse und Unterklasse sowie z. B. durch ihr elektrophoretisches Verhalten charakterisiert werden.

In der Diagnostik werden bevorzugt Antikörper in dem Fachmann an sich bekannten immunchemischen heterogenen oder homogenen Bestimmungsmethoden eingesetzt, wobei bei den homogenen Verfahren die Partikel-verstärkte Nephelometrie oder Turbidimetrie bevorzugt ist. Bei den heterogenen Immunoassays wird der festphasengebundene Sandwichassay bevorzugt, wobei die Festphase bevorzugterweise ein Polystyrolröhrchen, eine Mikrotitrationsplatte, ein Latexpartikel, ein magnetisierbarer Partikel oder eine flächenförmige Festphase ist.
Besonders bevorzugt ist der festphasengebundene Sandwichassay, wobei als Festphase eine Mikrotitrationsplatte eingesetzt wird.

Bevorzugt ist auch ein diagnostisches Verfahren zum Nachweis von Plasmin-Antiplasmin Komplex, wobei mindestens ein erfindungsgemäßer Antikörper als spezifischer Bindungspartner eingesetzt wird.

Der zweite spezifische Bindungspartner kann ein weiterer Antikörper oder ein Antikörperfragment, ein Lectin oder ein Rezeptor sein. Bevorzugt ist dabei ein Verfahren, bei dem auch der zweite spezifische Bindungspartner ein erfindungsgemäßer monoklonaler Antikörper ist, der ein anderes Epitop erkennt, als der erste spezifische Bindungspartner; dadurch lassen sich Einschritt-Assays realisieren.

Wird nach der Bindungsreaktion ein Waschschritt vorgenommen, bei dem der an den Fänger-Antikörper gebundene Komplex von dem flüssigen Überstand abgetrennt wird, so kann als zweiter spezifischer Bindungspartner auch ein Antikörper gegen eine der Einzelkomponenten des Komplexes eingesetzt werden; dieser kann auch polyklonal sein.

Zum Nachweis und zur Quantifizierung kann dabei einer der spezifischen Bindungspartner eine nachweisbare Markierung tragen. Diese Markierung ist dem Fachmann an sich bekannt und kann z. B. ein Chromophor, ein Luminophor, ein Fluorophor, ein Enzym, ein radioaktives Isotop oder ein gefärbter oder auch ungefärbter Partikel sein.
Zur Herstellung Antikörper-beschichteter Festphasen sind Verfahren bevorzugt, die den nicht-markierten spezifischen Bindungspartner nach dem Fachmann an sich bekannten Verfahren direkt oder indirekt, z. B. über einen weiteren Antikörper oder eine Biotin-Avidin Brücke, die an eine Festphase gekoppelt ist, binden.

Biosensoren, die unter Verwendung eines spezifischen Antikörpers zum Nachweis von Proteinen dienen, sind dem Fachmann an sich bekannt. Die erfindungsgemäßen Antikörper können auch in solchen Biosensoren eingesetzt werden.

Die erfindungsgemäßen Antikörper können auch nach dem Fachmann bekannten Verfahren z. B. radioaktiv markiert werden, um sie für die Immunszintigraphie einzusetzen.

Besonders bevorzugt sind die in dem Beispiel beschriebenen Ausführungsformen.

Beispiel und Ansprüche sind Bestandteil der Offenbarung.

Das folgende Beispiel erläutert die Erfindung, schränkt sie aber in keiner Weise ein:

### Beispiel 1:

### Schritt 1:

### Herstellung von Plasmin-inaktiviertem α2-Antiplasmin

2.3 mg α2-Antiplasmin (Fa. Biopool) in 1.2 ml Puffer A (0.1 mol/L KH₂PO₄, 0.15 mol/L NaCl) wurden auf Plasmin-Fractogel (24 mg Plasmin - Fa. Kabi - gekoppelt an 4 ml CNBr-Fractogel^{(R)}) aufgetragen und 1 h bei 25 °C inkubiert, um die Bildung eines Pl-AP-Komplexes zu ermöglichen. Das Gel wurde mit 40 ml Puffer A gewaschen. Anschließend wurde das Gel mit 0.25 % Ammoniak gespült, um die gegen nucleophile Agenzien unbeständige Bindung zwischen Enzym und Inhibitor zu hydrolysieren. Die proteinhaltigen Fraktionen des Ammoniak-Eluats enthielten 1.3 mg Plasmin-inaktiviertes α2-Antiplasmin.

### Schritt 2:

### Herstellung und Reinigung monoklonaler Antikörper gegen Pl-AP

### 2.1. Gewinnung Antikörper-produzierender Zellen

BALB/c-Mäuse wurden durch subkutane Injektion einer Emulsion von 50 µg Plasmin in komplettem Freund'schem Adjuvans immunisiert (Tag 1). An den Tagen 28 und 56 wurden je 50 µg Plasmin emulgiert in inkomplettem Freund'schem Adjuvans ebenfalls subkutan injiziert. Am Tag 92 folgte eine intraperitoneale Injektion von 100 µg Plasmin in 0.5 ml physiologischer Kochsalzlösung. Am Tag 95 wurden nach Entnahme der Milz durch mechanische Desintegration Lymphozyten gewonnen. Auf die gleiche Weise wurden Lymphozyten aus Tieren gewonnen, die mit Plasmininaktiviertem α2-Antiplasmin (hergestellt wie in Schritt 1 beschrieben), immunisiert wurden.

### 2.2. Fusion von Lymphozyten mit Myelomzellen

Hybridomazellen wurden nach Standardverfahren (Köhler und Milstein, 1975; Nature 256: 495 - 497) gewonnen: Die Myelomzellinie SP2/0-Ag14 wurde in Dulbecco's Modified Eagle's Medium (DMEM) mit 10 % foetalem Kälberserum (FKS) kultiviert. Für eine Fusion zur Erzeugung von Hybridomazellen wurden die Milzzellen einer Maus (ca. 10⁸ Lymphozyten) mit 5 x 10⁷ Myelomzellen gemischt, in serumfreiem DMEM gewaschen und abzentrifugiert. Nach vollständiger Entfernung des Überstands wurden dem Zellpellet 0.5 ml einer 50 %igen Lösung von Polyethylenglykol 4000 in DMEM innerhalb einer Minute zugetropft. Die Suspension wurde 90 Sekunden bei 37 °C inkubiert und anschließend durch Zugabe von 7.5 ml DMEM über einen Zeitraum von 5 Minuten verdünnt. Nach einer Inkubation von 10 Minuten bei Raumtemperatur wurde mit DMEM auf 40 ml aufgefüllt, und die Zellen wurden abzentrifugiert. Nach Absaugen des Überstands wurden die Zellen in DMEM mit 20 % FKS resuspendiert und auf 6 Mikrotiterplatten ausgesät (200 µl pro Kavität). Die Selektion von Hybridomazellen erfolgte durch Zusatz von 13.6 mg/ml Hypoxanthin, 0.18 mg/ml Aminopterin, 3.9 mg/ml Thymidin (HAT-Medium). Verbrauchtes Medium wurde in Abständen von 3 - 4 Tagen durch frisches ersetzt, nach 10 Tagen wurde HAT-Medium durch HT-Medium ersetzt.

### 2.3. Test auf Pl-AP-Antikörper

14 Tage nach der Fusion wurden die Zellkulturüberstände mittels Enzymimmunoassay auf Antikörper gegen den Pl-AP-Komplex getestet. Polystyrol-Mikrotestplatten wurden mit 3 µg/ml Kaninchen-anti-Maus-IgG in 0.1 mol/L NaHCO₃, pH 9.6, inkubiert (24 h, 4 °C). Anschließend wurden Zellkulturüberstände appliziert (2 h, 37 °C), gefolgt von einer Inkubation mit Standard-Humanplasma (1:2 verdünnt).

Danach folgte eine Inkubation (2h, 37 °C) mit einer Mischung aus Peroxidase-markierten Antikörpern aus Kaninchen gegen Plasminogen und α2-Antiplasmin (jeweils 1 µg/ml). Als substrat wurde eine Lösung von 0.1 % (Gew./Vol.) 2,2'-Azino-di-(3-ethylbenzthiazolinsulfonat) und 0.012 % (Vol./Vol.) H₂O₂ in 0.1 mol/L Citronensäure, 0.1 mol/L Na₂HPO₄, pH 4.5 verwendet. Nach einer Inkubation von 30 min bei 37 °C wurde die Absorption bei 405 nm gemessen. Zwischen den einzelnen Inkubationsschritten wurde mit 0.01 mol/L Na₂HPO₄, 0.01 mol/L NaH₂PO₄, pH 7.2, 0.15 mol/L NaCl, 0.05 % Tween® 20 (PBS/Tween) gewaschen. Alle Reagenzien wurden in PBS/Tween mit 2 % Rinderserumalbumin verdünnt. Parallel wurde mit denselben Zellkulturüberständen ein Enzymimmunoassay durchgeführt, bei dem anstelle von Standard-Humanplasma ein Plasma mit einem hohen Gehalt an Pl-AP-Komplex (gewonnen durch Inkubation von Standard-Humanplasma mit immobilisierter Urokinase) eingesetzt wurde. Antikörper, die mit dem Pl-AP-Komplex reagieren (Reaktion mit Pl-AP-haltigem Plasma), aber freies Plasminogen oder α2-Antiplasmin im Plasma nicht erkennen (keine Reaktion mit Standard-Humanplasma), sind zur Verwendung in einem ELISA für den Pl-AP-Komplex besonders geeignet.

### 2.4. Klonierung von AntikörperAntikörper-produzierenden Zellinien

Zellen, deren Überstände im Test auf Pl-AP-spezifische Antikörper positiv reagierten, wurden nach der Methode der limitierenden Verdünnung kloniert. Dazu wurden ca. 60 Zellen in DMEM mit 20 % FKS und 5 % Human Endothelial Culture Supernatant (Costar) auf 96 Kavitäten einer Zellkulturplatte verteilt. Einzelklone wurden mikroskopisch identifiziert und auf Antikörperproduktion wie oben beschrieben getestet. Die Klonierung wurde zweimal wiederholt.

### 2.5. Reinigung von monoklonalen Antikörpern

Klonale Zellinien wurden zur Produktion von Antikörpern in Roller-Flaschen überführt und in Iscove's Modified Dulbecco's Medium kultiviert. Zellüberstand wurde durch Zentrifugation gewonnen und mit Hilfe von Ultrafiltration etwa 10fach konzentriert. Das Konzentrat wurde über Protein A-Sepharose^{(R)}CL-48 (Pharmacia) gegeben, gebundenes IgG wurde mit 0.2 mol/L Glycin/ HCl, pH 3.0 eluiert. Die proteinhaltigen Fraktionen wurden gegen 0.1 mol/L Citrat, pH 6.5, dialysiert und mit Ultrafiltration auf ca. 5 mg/ml konzentriert.

### Schritt 3:

### Testen der gereinigten Antikörper

### 3.1. Herstellen von Antikörper-beschichteten Polystyrol-Mikrotitrationsplatten:

Die nach Schritt 2.5. gewonnenen Pl-AP-spezifischen Antikörper wurden mit Natriumphosphat-Pufferlösung (0.01 mol/L, pH 6.8) auf eine Konzentration von 5 µg/ml verdünnt und durch Adsorption an Mikrotitrationsplatten immobilisiert. Pro Vertiefung wurden 150 µl Antikörperlösung für 20 Stunden bei 20 °C inkubiert, anschließend wurde die Flüssigkeit abgesaugt und die Platten luftdicht verschlossen bei 4 °C gelagert.

### 3.2. Durchführung des Enzymimmunoassays (ELISA)

Die zu testenden Proben wurden mit Inkubationspuffer (0.1 mol/L NaCl, 0.1 mol/L Tris, 1 % Tween® 20, 0,1 % NaN₃, pH 7.2) 1+1 verdünnt. Jeweils 100 µl pro Vertiefung wurden 30 min bei 37 °C inkubiert. Anschließend wurde die Inkutionslösung entfernt und die Vertiefungen zweimal mit je 150 µl Waschlösung (0.02 mol/L Natriumphosphat, 0.05 % Tween® 20, pH 7.6) gewaschen. Anschließend wurden je 100 µl Peroxidase-konjugierte Kaninchen-anti-Plasminogen-Antikörper zugefügt und 30 min bei 37 °C inkubiert. Nach Entfernen der Konjugat-Lösung und zweimaligem Waschen wurden 100 µl Substrat-Lösung (Wasserstoffperoxid; o-Phenylendiamin) zugefügt und die Platte bei Raumtemperatur inkubiert. Nach 30 min Inkubation wurde die Peroxidase mit 0.1 mol/L Schwefelsäure inaktiviert und die Extinktion der Reaktionslösung bei 492 nm bestimmt. In Tabelle 1 sind die Extinktionswerte (OD 492 nm/30 min) von unterschiedlichen Konzentrationen an Pl-AP-Komplex dargestellt. Als Beschichtungsantikörper für die Mikrotitrationsplatten wurde sowohl ein monoklonaler Antikörper gegen ein Pl-AP-Neoantigen (PAP 6) als auch ein monoklonaler Antikörper gegen α2-Antiplasmin (APl 2) eingesetzt. In Tabelle 1 sind die Extinktionswerte und die gemessenen Konzentrationen dargestellt:

**Tabelle 1**

| | Beschichtungsantikörper | |
|---|---|---|
| | PAP 6 | APl 2 |
| Pl-AP-Komplex (ng/ml) | OD 492 nm | OD 492 nm |
| 3 | 0.060 | 0.040 |
| 10 | 0.066 | 0.040 |
| 30 | 0.102 | 0.060 |
| 100 | 0.211 | 0.120 |
| 300 | 0.490 | 0.300 |
| 1000 | 1.049 | 0.715 |
| 3000 | 1.678 | 1.375 |

Die Ergebnisse zeigen, daß grundsätzlich sowohl Antikörper gegen ein Pl-AP-Neoantigen als auch Antikörper gegen α2-Antiplasmin als Beschichtungsantikörper zur Bestimmung von Pl-AP-Komplex verwendet werden können.

### 3.3. Wiederfindung von Pl-AP-Komplex in Plasma

Einer Plasmaprobe (Normalplasma) wurden definierte Mengen an Pl-AP-Komplex zugesetzt und mittels ELISA bestimmt. Als Beschichtungsantikörper für Mikrotitrationsplatten wurde sowohl PAP 6 als auch APl 2 eingesetzt. In Tabelle 2 sind die Extinktionswerte dargestellt:

**Tabelle 2:**

| | Beschichtungsantikörper | |
|---|---|---|
| | PAP 6 | APl 2 |
| Pl-AP-Komplex (ng/ml) | OD 492 nm | OD 492 nm |
| 30 | 0.033 | 0.003 |
| 100 | 0.086 | 0.004 |
| 300 | 0.237 | 0.013 |

Die Ergebnisse zeigen eindeutig, daß mit dem Fängerantikörper PAP 6, der gegen ein Neoantigen auf dem Pl-AP-Komplex gerichtet ist, in der 1:2 verdünnten Plasmaprobe Pl-AP nachweisbar ist, während mit dem Antikörper APl 2 (gegen α2-Antiplasmin gerichtet) aufgrund der Kompetition von freiem α2-Antiplasmin und Pl-AP-Komplex Pl-AP erst in hohen Konzentrationen gefunden wird.

### 3.4. Detektion von Pl-AP-Komplex in Plasma

Plasma wurde mit Urokinase versetzt und 180 min bei 37 °C inkubiert. Zu verschiedenen Zeiten wurden Aliquote entnommen und die Reaktion durch Zugabe von Aprotinin abgestoppt. Die Proben wurden 1+99 mit PBS/Aprotinin verdünnt und mittels ELISA getestet. Zur Beschichtung der Mikrotitrationsplatten wurde der monoklonale Antikörper BMA AP6 (gegen Pl-AP-Neoantigen) verwendet. Tabelle 3 gibt die Ergebnisse wieder.

**Tabelle 3**

| Zeit (min) | OD 492 nm |
|---|---|
| 0 | <0.1 |
| 15 | 0.1 |
| 30 | 0.18 |
| 60 | 0.24 |
| 120 | 0.37 |
| 180 | 0.56 |

Die Ergebnisse zeigen, daß die Konzentration des Pl-AP-Komplexes im Plasma im Verlauf der Plasminogenaktivierung durch Urokinase mit zunehmender Zeit ansteigt.

## Patentansprüche (Patentansprüche für folgende(n) Vertragsstaat(en): AT, BE, CH, DE, DK, FR, GB, IT, LU, NL, PT, SE, LI)

1. Monoklonale Antikörper gegen den Plasmin-Antiplasmin Komplex, dadurch gekennzeichnet, daß sie eine hohe spezifische Affinität zu dem Komplex aufweisen und keine oder nur eine sehr geringe Affinität zu den freien Einzelkomponenten bzw. Vorläufern Plasminogen und Antiplasmin haben.

2. Antikörper gemäß Anspruch 1, dadurch gekennzeichnet, daß er von der Hybridomazellinien BW PAP6 (DSM ACC 2004) produziert wird.

3. Antikörper gemäß Anspruch 1, dadurch gekennzeichnet, daß sie an ein Antigen binden, das durch den Referenzantikörper BMA PAP6 gebunden wird.

4. Antikörper gemäß Anspruch 1, dadurch gekennzeichnet, daß es der MAK, BMA PAP6 ist.

5. Antigen, dadurch gekennzeichnet, daß es von einem Antikörper nach mindestens einem der Ansprüche 1 - 4 gebunden wird.

6. Hybridomazellinie, dadurch gekennzeichnet, daβ sie monoklonale Antikörper gemäß Anspruch 1 produziert.

7. Hybridomazellinie, dadurch gekennzeichnet, daß sie MAK BMA PAP6 produziert.

8. Hybridomazellinie; dadurch gekennzeichnet, daß sie die Zellinie BW PAP 6 (DSM ACC 2004) ist.

9. Verwendung eines Antikörpers gemäß Anspruch 1 zur Diagnostik; in vitro.

10. Diagnostisches in vitro Verfahren zum Nachweis des Plasmin-Antiplasmin Komplex, dadurch gekennzeichnet, daß mindestens ein Antikörper gemäß Anspruch 1 als spezifischer Bindungspartner eingesetzt wird.

11. Verfahren gemäß Anspruch 10, dadurch gekennzeichnet, daß mindestens einer der spezifischen Bindungspartner mit einer nachweisbaren Markierung versehen ist.

12. Verfahren gemäß Anspruch 11, dadurch gekennzeichnet, daβ die Markierung z. B. ein Enzym ist und der andere spezifische Bindungspartner direkt oder indirekt an eine Festphase gebunden wird.

## Patentansprüche (Patentansprüche für folgende(n) Vertragsstaat(en): ES)

1. Verfahren zur Herstellung monoklonales Antikörper durch Immunisierung von Säugetieren und Immortalisierung von Milzzellen der immunisierten Säugetiere durch Fusion mit einer immortalisierten Zellinie und Auswahl von Hybridomazellinien der gewünschten Spezifität, dadurch gekennzeichnet, daß die Antikörper gegen den Plasmin-Antiplasmin Komplex gerichtet sind und sie eine hohe spezifische Affinität zu dem Komplex aufweisen und keine oder nur eine sehr geringe Affinität zu den freien Einzelkomponenten bzw. Vorläufern Plasminogen und Antiplasmin haben.

2. Verfahren gemäß Anspruch 1, dadurch gekennzeichnet, daß die Hybridomazellinie die Zellinie BW PAP6 (DSM ACC 2004) ist.

3. Verfahren gemäß Anspruch 1, dadurch gekennzeichnet, daß der produzierte Antikörper an ein Antigen bindet, das durch den Referenzantikörper BMA PAP6 gebunden wird.

4. Verfahren gemäß Anspruch 1, dadurch gekennzeichnet, daß der produzierte Antikörper der MAK BMA PAP6 ist.

5. Verwendung eines Antikörpers der nach dem Verfahren gemäß Anspruch 1 produziert wurde zur Diagnostik, in vitro.

6. Diagnostisches in vitro Verfahren zum Nachweis des Plasmin-Antiplasmin Komplex, dadurch gekennzeichnet, daß mindestens ein Antikörper, der gemäß Anspruch 1 produziert wurde, als spezifischer Bindungspartner eingesetzt wird.

7. Verfahren gemäß Anspruch 6, dadurch gekennzeichnet, daß mindestens einer der spezifischen Bindungspartner mit einer nachweisbaren Markierung versehen ist.

8. Verfahren gemäß Anspruch 7, dadurch gekennzeichnet, daß die Markierung ein Enzym ist und der andere spezifische Bindungspartner direkt oder indirekt an eine Festphase gebunden wird.

## Claims (Claims for the following Contracting State(s): AT, BE, CH, DE, DK, FR, GB, IT, LU, NL, PT, SE, LI)

1. A monoclonal antibody against the plasminantiplasmin complex, which has a high specific affinity for the complex and no affinity or only a very low affinity for the individual free components or precursors plasminogen and antiplasmin.

2. An antibody as claimed in claim 1, which is produced by the hybridoma cell lines BW PAP6 (DSM ACC 2004).

3. An antibody as claimed in claim 1, which binds to an antigen which is bound by the reference antibody BMA PAP6.

4. An antibody as claimed in claim 1, which is the MAb BMA PAP6.

5. An antigen which is bound by an antibody as claimed in at least one of claims 1 - 4.

6. A hybridoma cell line which produces the monoclonal antibody as claimed in claim 1.

7. A hybridoma cell line which produces MAb BMA PAP6.

8. A hybridoma cell line which is the BW PAP 6 (DSM ACC 2004) cell line.

9. The use of an antibody as claimed in claim 1 for diagnosis, in vitro.

10. A diagnostic in vitro method for the detection of the plasmin-antiplasmin complex, wherein at least one antibody as claimed in claim 1 is employed as specific binding partner.

11. The method as claimed in claim 10, wherein at least one of the specific binding partners is provided with a detectable label.

12. The method as claimed in claim 11, wherein the label is, for example, an enzyme, and the other specific binding partner is bound directly or indirectly to a solid phase.

## Claims (Claims for the following Contracting State(s): ES)

1. A process for the production of monoclonal antibodies by immunization of mammals and immortalization of spleen cells from the immunized mammals by fusion with an immortalized cell line and selection of hybridoma cell lines of the required specificity, wherein the antibodies are directed against the plasmin-antiplasmin complex, and they have a high specific affinity for the complex and have no, or only a very low, affinity for the free individual components or precursors plasminogen and antiplasmin.

2. The process as claimed in claim 1, wherein the hybridoma cell line is the BW PAP6 (DSM ACC 2004) cell line.

3. The process as claimed in claim 1, wherein the antibody produced binds to an antigen which is bound by the reference antibody BMA PAP6.

4. The process as claimed in claim 1, wherein the antibody produced is the MAb BMA PAP6.

5. The use of an antibody produced by the process as claimed in claim 1 for diagnosis, in vitro.

6. A diagnostic in vitro method for the detection of the plasmin-antiplasmin complex, wherein at least one antibody produced as claimed in claim 1 is employed as specific binding partner.

7. The method as claimed in claim 6, wherein at least one of the specific binding partners is provided with a detectable label.

8. The method as claimed in claim 7, wherein the label is an enzyme, and the other specific binding partner is bound directly or indirectly to a solid phase.

## Revendications (Revendications pour l'(les) Etat(s) contractant(s) suivant(s): AT, BE, CH, DE, DK, FR, GB, IT, LU, NL, PT, SE, LI)

1. Anticorps monoclonaux dirigés contre le complexe plasmine-antiplasmine, caractérisés en ce qu'ils présentent une haute affinité spécifique pour le complexe et n'ont pas d'affinité ou n'ont qu'une affinité très faible pour les composants individuels libres ou précurseurs plasminogène et antiplasmine.

2. Anticorps selon la revendication 1, caractérisé en ce qu'il est produit par la lignée d'hybridomes BW PAP6 (DSM ACC 2004).

3. Anticorps selon la revendication 1, caractérisé en ce qu'il se lie à un antigène qui est fixé par l'anticorps de référence BMA PAP6.

4. Anticorps selon la revendication 1, caractérisé en ce qu'il est l'AcM BMA PAP6.

5. Antigène, caractérisé en ce qu'il est fixé par un anticorps selon au moins l'une des revendications 1 à 4.

6. Lignée d'hybridomes, caractérisée en ce qu'elle produit des anticorps monoclonaux selon la revendication 1.

7. Lignée d'hybridomes, caractérisée en ce qu'elle produit l'AcM BMA PAP6.

8. Lignée d'hybridomes, caractérisée en ce qu'elle est la lignée cellulaire BW PAP6 (DSM ACC 2004).

9. Utilisation d'un anticorps selon la revendication 1, pour le diagnostic in vitro.

10. Procédé de diagnostic in vitro pour la détection du complexe plasmine-antiplasmine, caractérisé en ce que l'on utilise comme partenaire de liaison spécifique au moins un anticorps selon la revendication 1.

11. Procédé selon la revendication 10, caractérisé en ce qu'au moins l'un des partenaires de liaison spécifiques est muni d'un marqueur détectable.

12. Procédé selon la revendication 11, caractérisé en ce que le marqueur est par exemple une enzyme et l'autre partenaire de liaison spécifique est lié directement ou indirectement à une phase solide.

## Revendications (Revendications pour l'(les) Etat(s) contractant(s) suivant(s): ES)

1. Procédé pour la production d'un anticorps monoclonal par immunisation de mammifères et immortalisation de cellules spléniques des mammifères immunisés, par fusion avec une lignée cellulaire immortalisée et sélection de lignées d'hybridomes ayant la spécificité désirée, caractérisé en ce que les anticorps sont dirigés contre le complexe plasmine-antiplasmine et présentent une haute affinité spécifique pour le complexe, et n'ont pas d'affinité ou n'ont qu'une affinité très faible pour les composants individuels libres ou précurseurs plasminogène et antiplasmine.

2. Procédé selon la revendication 1, caractérisé en ce que la lignée d'hybridomes est la lignée cellulaire BW PAP6 (DSM ACC 2004).

3. Procédé selon la revendication 1, caractérisé en ce que l'anticorps produit se lie à un antigène qui est fixé par l'anticorps de référence BMA PAP6.

4. Procédé selon la revendication 1, caractérisé en ce que l'anticorps produit est l'AcM BMA PAP6.

5. Utilisation d'un anticorps qui a été produit selon le procédé conforme à la revendication 1, pour le diagnostic in vitro.

6. Procédé de diagnostic in vitro pour la détection du complexe plasmine-antiplasmine, caractérisé en ce que l'on utilise comme partenaire de liaison spécifique au moins un anticorps qui a été produit selon la revendication 1.

7. Procédé selon la revendication 6, caractérisé en ce qu'au moins l'un des partenaires de liaison spécifique est muni d'un marqueur détectable.

8. Procédé selon la revendication 7, caractérisé en ce que le marqueur est une enzyme et l'autre partenaire de liaison spécifique est lié directement ou indirectement à une phase solide.
